(19) Europäisches Patentamt
European Patent Office
Office européen des brevets

(11) EP 0 623 174 B1

(12) EUROPEAN PATENT SPECIFICATION

(45) Date of publication and mention of the grant of the patent:
**04.09.1996 Bulletin 1996/36**

(21) Application number: **93902437.8**

(22) Date of filing: **22.01.1993**

(51) Int Cl.[6]: **C12Q 1/34**, G01N 33/53, C12Q 1/48, C12Q 1/66, G01N 33/58, C07H 15/14, C07D 307/32

(86) International application number:
**PCT/GB93/00138**

(87) International publication number:
**WO 93/15220 (05.08.1993 Gazette 1993/19)**

# (54) HOMOCYSTEINE ASSAY

TESTVERFAHREN FÜR HOMOCYSTEINE

DOSAGE D'HOMOCYSTEINE

(84) Designated Contracting States:
**AT BE CH DE DK ES FR GB GR IE IT LI LU MC NL PT SE**

(30) Priority: **22.01.1992 NO 920282**
**10.02.1992 US 833118**
**06.03.1992 GB 9204922**

(43) Date of publication of application:
**09.11.1994 Bulletin 1994/45**

(60) Divisional application: **96200534.4**

(73) Proprietor: **AXIS BIOCHEMICALS AS**
**0505 Oslo (NO)**

(72) Inventor: **SUNDREHAGEN, Erling**
**N-1531 Moss (NO)**

(74) Representative: **Cockbain, Julian, Dr. et al**
**Frank B. Dehn & Co.,**
**European Patent Attorneys,**
**179 Queen Victoria Street**
**London EC4V 4EL (GB)**

(56) References cited:
**EP-A- 0 070 033** **FR-A- 2 549 853**

- **H.U. BERGMEYER (ed.), "Methods of Enzymatic Analysis", 1985, VCH VerlagesgesmbH, Weinheim (DE), 3rd edition, vol. VII; pp. 110-117; and pp. 357- 364; and pp. 403-409/**
- **CLINICAL CHEMISTRY, vol. 31, no. 4, April 1985, Winston, NC (US); H. REFSUM et al.; pp. 624-628**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 623 174 B1

Printed by Jouve, 75001 PARIS (FR)

## Description

The present invention relates to an assay for homocysteine in clinical samples.

Homocysteine is an intermediary amino acid produced when methionine is metabolised to cysteine. Generally, homocysteine produced in the body is rapidly metabolised by one of two routes, (1) condensation with serine to form cystathione or (2) conversion to methionine, and its concentration (and that of its oxidised form homocystine) in the living body under normal conditions is virtually negligible.

Homocysteine levels in biological samples may however be of clinical significance in a number of situations as homocysteine plays an important part in the complex set of pathways which make up sulphydryl amino acid metabolism and its accumulation may be indicative of various disorders occurring in these pathways, including in particular inborn errors of metabolism. Thus, for example homocystinuria (an abnormal build-up of homocysteine in the urine) is known to be a disorder of amino acid metabolism resulting from deficiencies -in the enzymes cystathione β synthetase or methyltetrahydrofolic acid methyltransferase (which catalyses the methylation of homocysteine to methionine).

Sulphydryl amino acid metabolism is closely linked to that of folic acid and vitamin $B_{12}$ (cobalamin), which act as substrates or co-factors in the various transformations involved. For this reason homocysteine accumulation has also been proposed as an indicator of malfunction of cobalamin or folate dependent enzymes, or other disorders or diseases related to cobalamin or folate metabolism.

Moreover since homocysteine conversion to methionine relies on a reaction requiring S-methyl tetrahydrofolate as the methyl donor, homocysteine metabolism may also be affected by anti-folate drugs, such as methotrexate, administered to combat other disorders, notably cancer. Monitoring of homocysteine has therefore also been proposed in the management of malignant disease treatment with anti-folate drugs.

More recently, elevated levels of homocysteine in the blood have been correlated with the development of atherosclerosis (see Clarke et al., New Eng. J. Med. 324:1149-1155 (1991)) and even moderate homocysteinemia is now regarded as a risk factor for cardiac and vascular diseases. Measurement of plasma or blood levels of homocysteine is thus also of importance in the diagnosis and treatment of vascular disease.

Although immunological methods of determining homocysteine directly are not available as there is no available antibody to homocysteine, a number of other methods for determining homocysteine in clinical samples have been proposed. These have all involved chromatographic separations and generally have been based on one of the three following principles:

(1) classical chromatographic amino acid analysis,

(2) reaction of homocysteine in the sample with the enzyme S-adenosyl-L-homocysteine hydrolase in the presence of a radioactively or otherwise labelled S-adenosine co-substrate followed by separation and quantitation of the product (S-adenosyl-L-homocysteine, SAH) formed. Generally chromatographic separation (HPLC or TLC) and radioactivity measurements are used (see Refsum et al., Clin. Chem. 31:624-628 (1985); Kredich et al., Anal. Biochem 116:503-510 (1981); Chui, Am. J. Clin. Path. 90(4):446-449 (1988); Totani et al., Biochem. Soc. 14(6): 1172-9 (1988); and Schimizu et al., Biotechnol. Appl. Biochem. 8:153-159 (1986)). An assay for SAH is also described in "Methods of Enzymatic Analysis", Bergmeyer et al. (Ed.), Third Edition, Vol. VII, VCH, Weinheim, 1985, pages 403-409.

(3) reaction of homocysteine in the sample with a fluorophore, followed by HPLC-separation and fluorometry (see Refsum et al., Clin. Chem. 35(9) ; 1921-1927 (1989)).

These methods are time-consuming and cumbersome to perform and all rely on direct quantitation. More particularly, chromatographic separation is a common feature of the prior art methods and requires highly specialised and sophisticated equipment.

The use of such equipment is generally not well accepted in routine clinical laboratory practice and such processes are consequently not generally amenable to automation in typical clinical laboratory procedures.

A need therefore exists for an improved assay for homocysteine which is simple, specific, quick to perform, readily adapted for use in clinical laboratories and above all which avoids the need for costly and time-consuming chromatographic separation. The present invention seeks to provide such an assay.

In one aspect the present invention therefore provides a method for assaying homocysteine in a sample, said method comprising the steps of (a) contacting said sample with the enzyme S-adenosyl homocysteine (SAH) hydrolase, and at least one of (i) S-adenosyl homocysteine and (ii) adenosine or an adenosine analogue, and (b) without chromatographic separation (i.e. of reagents or reaction products) assessing (preferably photometrically) a non-labelled analyte selected from adenosine, said adenosine analogue and S-adenosyl homocysteine.

After contacting the sample with the SAH hydrolase and adenosine, or the adenosine analogue, it is preferably

incubated for at least 30 seconds, especially at least 5 minutes before the subsequent stages of the assay are performed.

As used herein the term "assessing" is intended to include both quantitative and qualitative determination in the sense of obtaining an absolute value for the amount or concentration of the analyte present in the sample, and also obtaining an index, ratio, percentage, visual or other value indicative of the level of analyte in the sample. Assessment may be direct or indirect and the chemical species actually detected need not of course be the analyte itself but may for example be a derivative thereof or some further substance as discussed below.

The assay of the invention conveniently uses either enzymic or immunological techniques for analyte assessment. In one preferred enzymic technique the analyte is contacted with a further enzyme for which it is a substrate and either a co-substrate or a direct or indirect reaction product of the enzymic conversion of the analyte by that further enzyme is assessed. In a preferred immunological technique the analyte is assessed using a procedure involving competitive binding to an antibody by the analyte and a further hapten (e.g. a polyhapten or a labelled analogue of the analyte) and assessment of the bound or unbound hapten.

The homocysteine converting enzyme used according to the invention is S-adenosyl-homocysteine hydrolase (SAH-hydrolase) which catalyses the homocysteine reaction

$$\text{adenosine} + \text{homocysteine} \rightleftharpoons \underset{\text{(SAH)}}{\text{S-adenosyl-homocysteine}}$$

a reaction which has an equilibrium constant K of $10^6 M^{-1}$.

The reaction may run in either direction, depending on reaction conditions, reactant concentration etc.

In the above scheme, adenosine is the homocysteine co-substrate. Other co-substrates such as adenosine analogues or related compounds however may be used in the assay method of the invention.

The assay of the invention can take particular advantage of the fact that homocysteine acts as an inhibitor of SAH-hydrolase, suppressing the hydrolysis reaction which forms homocysteine and adenosine and pushing the reaction equilibrium in favour of SAH synthesis.

The amount of homocysteine in a sample thus indirectly influences the formation or consumption of the homocysteine co-substrate, e.g. adenosine, by SAH-hydrolase and thereby its resulting concentration in the reaction mixture. In this invention the resulting concentration, or change in the concentration of homocysteine co-substrate, e.g. adenosine, in the reaction mixture can be used as an indicator for the initial concentration of homocysteine in the sample. Thus where the analyte is the co-substrate the assay of the invention differs from prior art methods in that, rather than being assessed directly, homocysteine is assessed indirectly by determining the concentration of its co-substrate in its enzyme catalysed conversion. This has the direct advantage that detection methods which are suited to typical clinical laboratory procedures but which were not usable in the prior art assays for homocysteine, e.g. photometric methods, may be used so making the assay according to the invention particularly suited for routine clinical use.

In the assay method of the invention, the SAH-hydrolase reaction may be used in either direction. Thus if the test sample is contacted with adenosine and SAH-hydrolase, an amount of adenosine is consumed which corresponds to the amount of homocysteine consumed, and the amount of homocysteine in the sample can thus be determined from the alteration in the adenosine concentration. Adenosine analogues and/or adenosine generating compounds may be used in the place of adenosine itself.

In other preferred embodiments of the invention, the opposite direction of reaction may be used. The test sample may be contacted with SAH (generally in excess) and SAH-hydrolase. Homocysteine and adenosine are then formed from hydrolysis of the SAH. Any homocysteine present in the test sample will counteract this net reaction, and thus inhibit the formation of adenosine, the amount of which is monitored.

Clinical samples to be assayed according to the invention may be derived from any biological fluid or tissue extract and may be pretreated prior to assay. Plasma or urine samples will however generally be used.

In the plasma or urine, significant proportions of the homocysteine present may be bound by disulphide linkage to circulating proteins, such as albumin, and homocysteine may also be present in the form of other disulphide derivatives (generally homocysteine - cysteine conjugates). To obtain an estimate of total homocysteine present in the sample it may therefore be desirable to treat the sample with a reducing agent to cleave the disulphide bonds and liberate free homocysteine.

Disulphides are easily and specifically reduced by thiols (e.g. dithiothreitol (DTT), dithioerythritol (DTE), 2-mercapto-ethanol, cysteine-thioglycolate, thioglycolic acid, glutathione and similar compounds). Direct chemical reduction can be achieved using borohydrides (e.g. sodium borohydride) or amalgams (e.g. sodium amalgam) or more specialized reagents such as phosphines or phosphorothioates can be used. Disulphide reduction is reviewed by Jocelyn in Methods of Enzymology 143: 243-256 (1987) where a wide range of suitable reducing agents is listed.

Adenosine or the adenosine analogue may be assessed by known methods. Generally methods relying upon photometric (e.g. colorimetric, spectrophotometric or fluorometric) detection and immunological methods are preferred

as these may particularly readily be adapted for use in clinical laboratories. Methods based on enzymic reaction or reaction with mono- or polyclonal antibodies are particularly preferred, as these are simple and quick and can be relatively inexpensive to perform. Thus for example the analyte may be assessed by monitoring the reaction with enzymes which convert it directly or indirectly to products which may be detected photometrically, e.g. spectrophotometrically. Suitable enzymes, which should of course be non-reactive with the other substrates of SAH hydrolase; particularly homocysteine, include adenosine deaminase (which converts adenosine to inosine) and adenosine kinase (which converts adenosine and ATP to ADP and phosphorylated adenosine). Such enzymes may further be combined with other enzymes which act to convert the products formed to further detectable products.

Examples of immunological methods would include methods involving reaction of the analyte with antibodies specific for it which either are themselves assessable or which can be reacted further to form detectable products, eg. in a sandwich assay. One particularly attractive immunological method however involves the use of a fluorophore labelled analogue of the analyte, preferably a co-substrate, eg. fluorescein labelled adenosine - this and the unlabelled analyte may be contacted with an antibody for the analyte. If the resultant product is subjected to a fluorescence polarization assay using polarised exciting radiation an indication of the unlabelled analyte concentration may then be derived from the degree of depolarization of the fluorescence radiation. Adenosine antibodies are commercially available (eg. from Paessel & Lorei GmbH, Frankfurt, Germany and Serotech Ltd., Oxford, United Kingdom) and fluorescence polarization immunoassay (FPIA) techniques are well established (see for example US-A-4420568 and US-A-4593089 and other publications by Abbott Laboratories relating to their TDx technology).

Thus examples of detection schemes useful in the assay of the invention include

(1) adenosine + fluorescein labelled adenosine —(antibody)→ FPIA detection

(2) adenosine —(adenosine deaminase)→ inosine + $NH_3$

(3) adenosine —(adenosine deaminase)→ inosine —(purine nucleoside phosphorylase)→ hypoxanthine —($O_2$ → $H_2O_2$; Xanthine Oxidase)→ Xanthine —($O_2$ → $H_2O_2$; Xanthine Oxidase)→ Uric acid

(4)

$$\text{Adenosine} + \text{ATP} \xrightarrow{\text{adenosine kinase}} \text{Adenosine-5'-P} + \text{ADP}$$

together with a competing chemiluminescent ATP reaction, e.g.

$$\text{ATP} + \text{luciferin} + O_2 \xrightarrow{\text{luciferase}} \text{oxyluciferin} + PP_i + \text{AMP} + CO_2 + \text{light}$$

or with a fluorophore labelled adenosine which competes for the ATP/adenosine kinase, with assessment being performed by fluorescence polarization measurement.

As regards schemes (2) and (3), inosine and uric acid have distinctive UV absorption properties and can thus be monitored spectrophotometrically, by kinetic measurements.

However the use of UV detection of uric acid or inosine has certain limitations in that the sensitivity of the method is rather poor and it requires a UV-light source and a UV-transparent sample container. It may thus be more convenient to rely upon colorimetric detection or electronic sensors, and such methods, particularly colorimetry, are generally favoured in clinical laboratories.

In this connection the reaction of scheme (2) is particularly useful in that ammonia generated by the adenosine deaminase reaction may readily be detected using known colorimetric techniques. Thus for example ammonia generated in the sample may be reacted to form coloured products, the formation of which may be detected spectrophotometrically. One such method, described in Methods of Enzymatic Analysis (Bergmeyer) Volume 1:1049-1056 (1970) relies upon the reaction of ammonia with phenol in the presence of hypochlorite in alkaline conditions to form the coloured dye indophenol:

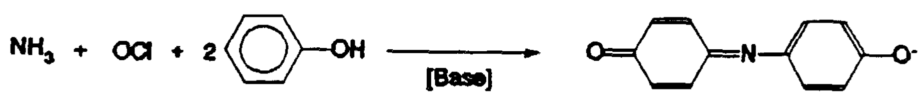


$Na_2[Fe(CN)_5NO]$

Sodium nitroprusside may be used as catalyst. Modifications of the method using for example various derivatives of phenol may also be used.

The coloured end-product is formed in amounts directly proportional to the concentration of ammonia, and hence adenosine, in the sample.

In scheme (3), the xanthine oxidase reaction lends itself to detection using fluorogens or chromogens, e.g. red-ox indicators, by assessing the reduction/oxidation potential, or by measuring $O_2$ consumption, or more particularly $H_2O_2$ formation, for example by the use of electronic sensors. Numerous red-ox indicators can be used for this purpose, and a wide range of methods are described in the literature for assaying $H_2O_2$ and $O_2$ in solution. Indeed, $H_2O_2$ is frequently detected in clinical assays.

Suitable red-ox indicators include methylene blue, 2,6-dichlorophenol, indophenol and the various red-ox indicators listed in Table 1 of the Kodak Laboratory & Research Products, Catalog No. 53, although others may of course be used. Enzymes with peroxidase activity, e.g. horseradish peroxidase, may be added to facilitate the red-ox reactions.

If a precipitating chromogen is desired, MTT tetrazolium may be used in combination with xanthine oxidase or other similar enzymes. By the use of a precipitating chromogen or fluorogen, a reading of immobilized colour or fluorescence can be obtained to obtain a visual indication of homocysteine concentration.

In scheme (4), a chemiluminescent ATP reaction is used to assess adenosine concentration. Chemiluminescence based assays have great potential due to the low detection limits achievable and to the relative simplicity of the necessary instrumentation.

Chemiluminescent reactions can be used to detect analytes such as ATP or $H_2O_2$ and one of the most efficient and best known such reactions is the firefly bioluminescence reaction

$$ATP + luciferin + O_2 \xrightarrow{\text{luciferase}} oxyluciferin + PP_i + AMP + CO_2 + light$$

Firefly luciferin has a benzothiazole structure but luciferins from other biological sources are available which have other structures. For analytical purposes ATP, luciferin or luciferase could be assayed directly using this reaction. Another chemiluminescent reaction which could be used where $H_2O_2$ is generated, as for example in scheme (3), is the luminol reaction (luminol is 5-amino-2,3-dihydro-phthalazine-1,4-dione) with hydrogen perioxidase catalyst which results in light emission at 425 nm.

Hydrogen peroxide, from scheme (3) for example, can also be assessed using the non enzymic chemiluminescent reactions of peroxioxalate and the acridinium esters, the latter in aqueous solution at neutral pH.

The use of fluorophore labelled adenosine in scheme (4) and assessment by fluorescence polarization measurement is feasible due to the relatively broad substrate specificity of adenosine kinase. This broad specificity can moreover be used to compensate for endogenous adenosine (or other adenosine kinease nucleoside substrates) by adding adenosine kinase to the sample as a pretreatment, preferably in combination with the reducing agent (e.g. DTT).

Such enzymic pretreatment of the sample is desirable as, in many of the embodiments of the invention, the analyte (e.g. adenosine) is already present in the sample in varying amounts, thus providing a potential source of error in the assay. Background analyte content can be compensated for by running the assay on a portion of the sample without using the homocysteine converting enzyme SAH-hydrolase; however such a procedure is time consuming and makes the assay more cumbersome. An alternative is pre-treatment of the sample with an agent serving to convert or remove the endogenous analyte, e.g. an enzyme such as adenosine kinase or adenosine deaminase which removes the backgound adenosine. As mentioned above, to avoid unnecessarily increasing the time required for the assay to be run, such treatment of the sample may conveniently be effected at the time that it is pre-treated with the reducing agent to liberate the homocysteine.

Besides the use of spectrometric or colorimetric techniques for analyte assessment, other photometric techniques may be used. Among the most useful techniques that can be used are particle agglutination and immunoprecipitation techniques. If polyclonal antibodies are used, direct particle agglutination or direct immunoprecipitation may be used, although this will generally not be preferred. However precipitation inhibition or particle agglutination inhibition techniques can be used. These rely on the use of antibody/hapten combinations which on conjugation lead to precipitation or particle aggregation which can be detected by turbidimetric or nephelometric measurement. Where the antibody/hapten complex formation is inhibited by the analyte, e.g. SAH, the SAH content may be assessed from the reduction in precipitation/aggregation. The reaction can be expressed as follows

Antibody (optionally particle-bound) + hapten (preferably a polyhapten)

→ complex formation (precipitation or particle aggregation)

It is advantageous either to store the SAH-hydrolase in the presence of a reducing agent or to treat it with a reducing agent prior to its use in the assay. It has been found that storage otherwise causes SAH-hydrolase to become inactivated and this use of a reducing agent either prevents inactivation during storage or causes reactivation prior to use.

Various reducing reagents can be used (for example DTT, cysteine, mercaptoethanol, dithioerythritol, sodium borohydride, etc.), however DTT is particularly suitable, e.g. at about 5mM concentration. DTT should itself be stored at low pH and thus the assay kit can conveniently include a solution of DTT at a low pH (e.g. about 3) but with a low buffer capacity and a separate solution of SAH-hydrolase, which may be partially or totally inactive, at substantially neutral pH and preferably buffered. When these solutions are combined, the enzyme is reactivated at neutral pH. This combination can if desired take place in the presence of the test sample, or with the test sample added shortly thereafter, so that the homocysteine liberation is effected simultaneously. The other reducing agents mentioned above may similarly be used for both SAH-hydrolase stabilization/activation and for reducing the sample to liberate homocysteine.

In the analytical kits for use in the method of the invention described below, reducing agents may be used to reactivate inactivated SAH-hydrolase. Thus such kits may comprise in a first compartment an inactive SAH-hydrolase and in a second compartment a reducing agent, e.g. DTT in an acid medium (for example pH 3). Using this kit the SAH-hydrolase can be mixed with the reducing agent, and so reactivated, immediately prior to its use in the assay.

Other additives may advantageously be used to enhance SAH-hydrolase stability during storage or in the assay itself. These include $NAD^+$, glutathione, polyhydric alcohols and sugars (e.g. inositol, sorbitol, xylitol, eryrthritol, glycerol, ethylene glycol, sucrose, lactitol, etc.), soluble polymers such as certain dextrans, and proteins (e.g. carrier proteins).

Where the assay of the invention uses antibodies, these may be polyclonal but preferably are monoclonal. Where

the desired antibodies are not already commercially available, they may be produced by standard techniques. Antibodies can thus be raised in animals or hybridomas, either monoclonal or polyclonal, e.g. as described by James Gooding in "Monoclonal antibodies, principle and practice", Academic Press, London, 1983, Chapter 3. Monoclones must be sorted to select clones which discriminate between the desired hapten and other substrates for the enzyme (s), e.g. which discriminate between adenosine and SAH. Polyclonal antibodies reactive only with the analyte (e.g. SAH) should be purified to remove cross-reacting antibodies, i.e. those reactive with other substrates besides the analyte, e.g. with both adenosine and SAH. This can be done by affinity chromatography, e.g. where SAH is the analyte by using immobilized adenosine.

In the production of the antibody one uses as a hapten either the analyte itself or another molecule which includes the portion of the analyte that is considered to be the most appropriate binding region, e.g. a region remote from those regions participating in the enzymic reaction. The hapten is conveniently conjugated to a macromolecule such as BSA or hemocyanin. For SAH, the desired epitope is preferably at or about the thioether bridge and thus while one can use SAH itself

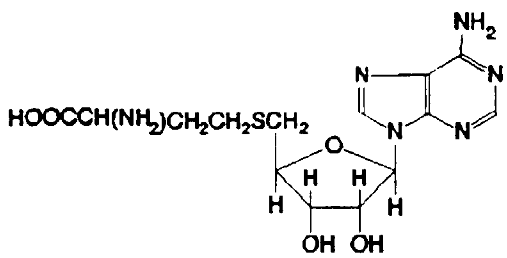

conjugated to a macromolecule, it is preferred to use a "simplified" molecule such as one of formula (I)

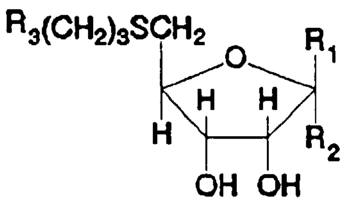

(I)

(where $R_1$ and $R_2$ which may be the same or different are hydrogen atoms or $OR_4$ groups (wherein $R_4$ is a lower (e.g. $C_{1-6}$, especially $C_{1-4}$) aliphatic group such as an alkyl group, preferably a methyl or ethyl group) or $R_1$ and $R_2$ together represent an oxygen atom, and $R_3$ is an amine or carboxyl moiety) or a salt or ester (e.g. with a $C_{1-4}$, alkanol) thereof, again coupled to a macromolecule.

Examples of compounds of formula I that may be used as haptens thus include

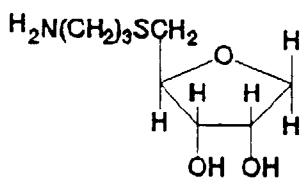

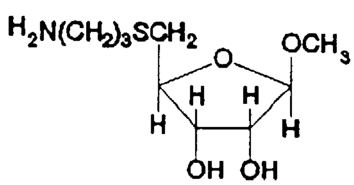

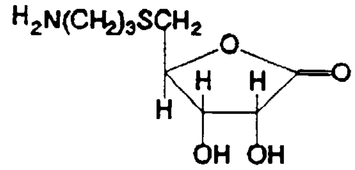

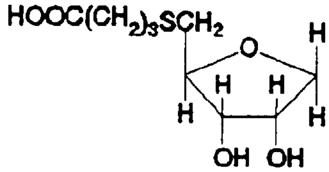

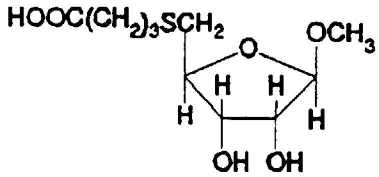

and

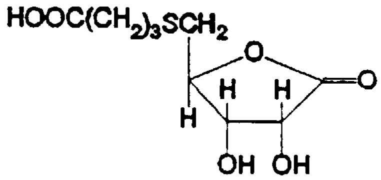

Such "simplified" structures may also be adopted for the labelled analogues mentioned above used in assays where the analyte and labelled analogue are involved in a competitive binding reaction with the antibody. Thus the signal giving moiety R*, which may be selected from fluorophores, chromophores, radiolabels, enzymic, chemiluminescent and other lables conventionally used in immunoassays can be conjugated to the analyte,
as for example R*-SAH,
or to a simplified, epitope containing molecule as for example

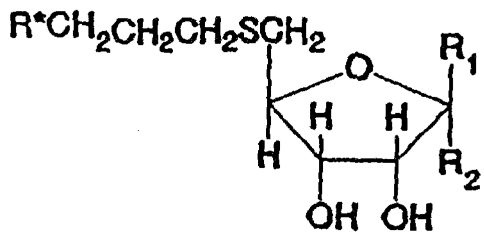

Such labelled moieties can of course be coupled to particles, polymers, proteins or other materials if desired.

The compounds of formula I may be prepared by a process comprising at least one of the following steps:

(a) reacting a compound of formula II

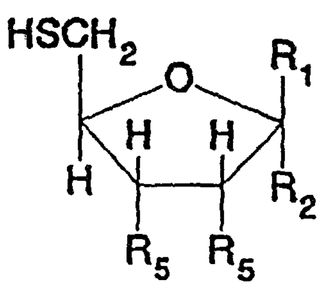

(II)

(wherein $R_1$ and $R_2$ are as defined above and each $R_5$ is a protected hydroxyl group or both $R_5$ together are an

alkylenedioxy group (i.e. a protected bis hydroxy group, such as -$OC(CH_3)_2O$-) with a propyl halide of formula III

$$R_6(CH_2)_3\ Hal \qquad (III)$$

(where $R_6$ is an $R_3$ group or a protected $R_3$ group and Hal is a halogen atom, e.g. a bromine atom) followed by removal of any protecting groups if desired;

(b) (to produce a compound of formula I wherein $R_3$ is an amino group) reacting a compound of formula II with acrylonitrile and reducing and deprotecting the cyano propyl thio-ether product obtained; and

(c) esterifying a compound of formula I wherein $R_3$ is a carboxyl group.

The starting products of formula III can be produced by standard techniques or are known from the literature. The starting products of formula II may be produced from the corresponding 1-hydroxymethyl furanoses by cis-hydroxy group protection, bromination and subsequent reaction with thiourea and hydrolysis. The 1-hydroxymethyl-furanoses may be cis-hydroxy group protected by reaction with conventional hydroxy protecting agents, e.g. acetone.

Examples of reaction schemes for the preparation of compounds of formula I include the following (compounds (1) and (3) are commercially available)

(A) 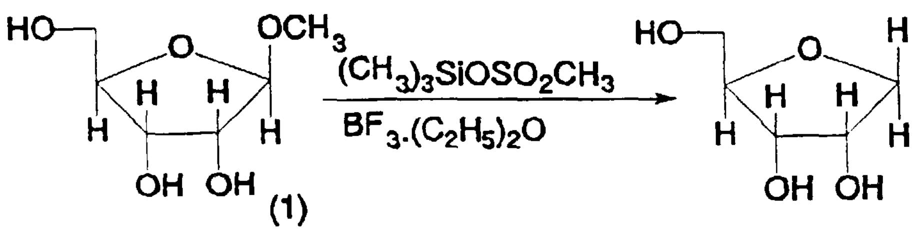


(1)

(2)

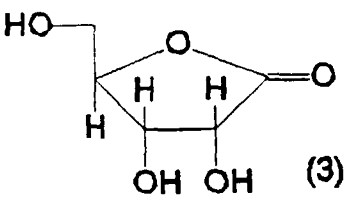

(3)

(B) 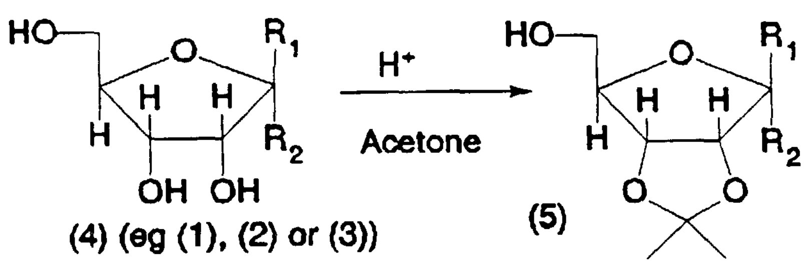


(4) (eg (1), (2) or (3))

(5)

(C) 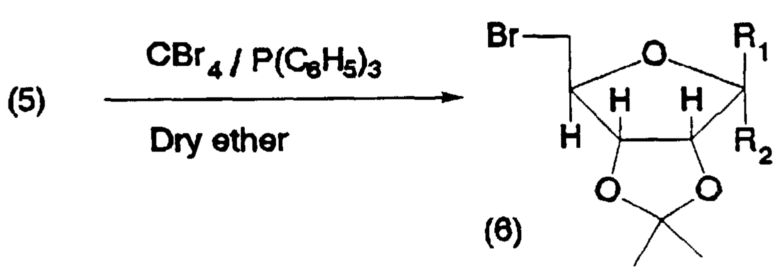


(5)

(6)

(D)

(6)

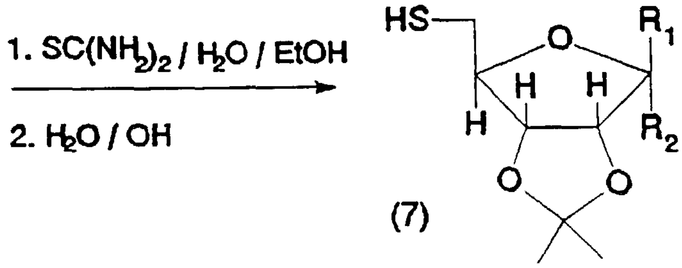


(7)

(E)

(7)

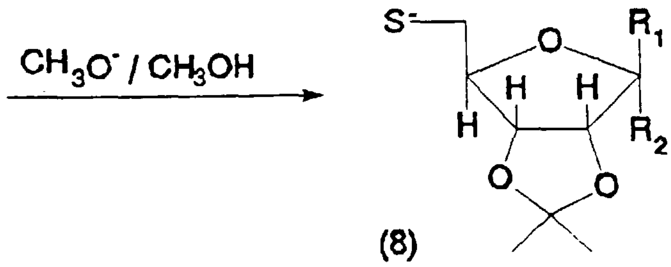


(8)

(F)

(8)

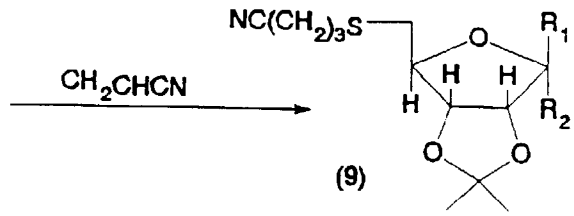


(9)

(G)

(9)

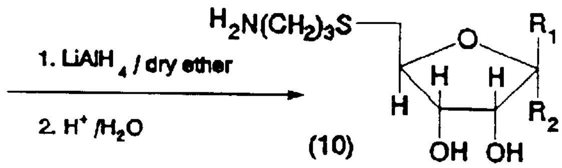


(10)

(H)

(8)

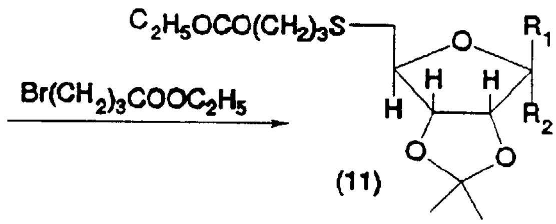


(11)

(I)

(11)

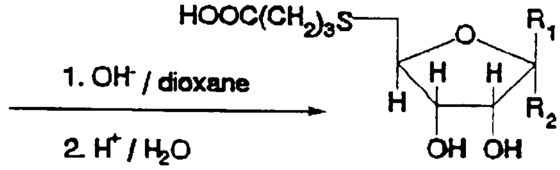


(12)

(J)

(12)

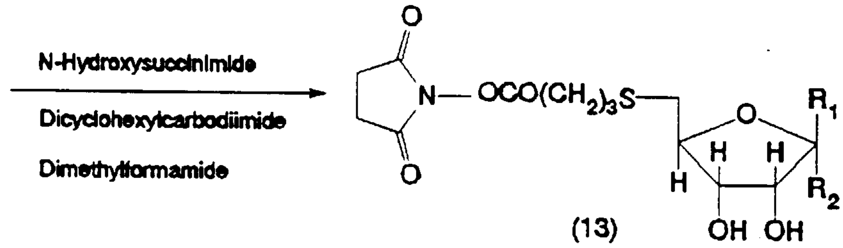


(13)

UV absorption, absorption of visible light, or fluorescence by substances in the reaction mixture, optionally precipitated or otherwise separated from the reaction mixture, may be measured at the reaction end point (when the signal is stable) or at one or more fixed time points or alternatively a kinetic measurement may be adopted in which several measurements are made at different points of time.

To perform the assay of the invention, the necessary reagents may be added to the reaction mixture in a sequential manner or simultaneously. However, in many preferred embodiments, one or more of the reactions may advantageously be run for some time before the addition of reagents for the subsequent reaction(s). As an example, where the test sample is reacted with adenosine and SAH hydrolase, the formation of SAH from homocysteine and adenosine is preferably allowed to take place for some time before the adenosine assessment procedure is initiated.

In clinical chemistry analysis, the use of standard curves for calibration purposes is standard practice. Thus, in the performance of the method of this invention, samples of known homocysteine content may be used in the place of clinical samples to construct a standard curve for the response/signal to be measured and the homocysteine content of the unknown samples may then be calculated by interpolation from the standard curve. Thus an exact quantification of the signal forming molecules or the red-ox potentials is not necessary.

The assay method of the present invention may be used for the diagnosis and monitoring of pathological or potentially pathological conditions which are related to or manifested in the homocysteine content of body fluids or tissues. These include atherosclerosis, blood diseases, vitamin deficiencies and/or inborn errors of metabolism. It may also be used for the evaluation of the effects of pharmaceuticals, such as anti-folate drugs.

In another aspect the invention provides an analytical kit for use in the assay of homocysteine in a sample, by the method of the invention, said kit comprising: SAH-hydrolase; a substrate selected from adenosine, adenosine analogues and S-adenosyl homocysteine; a signal forming agent; and, optionally, means for signal assessment.

In one preferred embodiment, the kit comprises: S-adenosyl-homocysteine hydrolase; one or more substrates selected from adenosine, adenosine analogues and S-adenosyl homocysteine; means for generating a detectable derivative of an analyte selected from adenosine, adenosine analogues and S-adenosyl homocysteine; and, optionally, means for spectrometrically or colorimetrically assessing said detectable derivative to provide an indication of the homocysteine content of the sample.

In a further embodiment the kit comprises S-adenosyl-homocysteine hydrolase; as a signal forming substrate for said enzyme, labelled S-adenosyl-homocysteine; an optionally carrier matrix bound anti-S-adenosyl homocysteine antibody; and, optionally, as said means for signal assessment, means for photometrically assessing said labelled S-adenosyl homocysteine or the detectable derivative thereof whereby to provide an indication of the homocysteine content of the sample.

In another embodiment, the kit comprises: adenosine; S-adenosyl-homocysteine hydrolase; an adenosine converting enzyme other than SAH-hydrolase; optionally, a co-substrate for said adenosine converting enzyme; and means for generating a photometrically detectable response from said co-substrate or from a product of enzymic conversion of adenosine by said adenosine converting enzyme.

Thus for example the adenosine converting enzyme may be adenosine kinase and the means for generating may comprise ATP, luciferin and a luciferase.

Alternatively, the adenosine converting enzyme may be adenosine deaminase and the means for converting may comprise nucleoside phosphorylase, xanthine oxidase and a peroxidase.

In a still further embodiment the kit comprises adenosine; S-adenosyl-homocysteine; an optionally matrix particle bound anti-S-adenosyl-homocysteine antibody; a polyhapten for said antibody; and, optionally, means for photometrically assessing agglutination or precipitation of antibody:polyhapten complexes. In this embodiment, the polyhapten may conveniently be provided by a backbone polymer to which are conjugated a plurality of furanose 6-thioethers, e.g. leaving pendent residues of formula

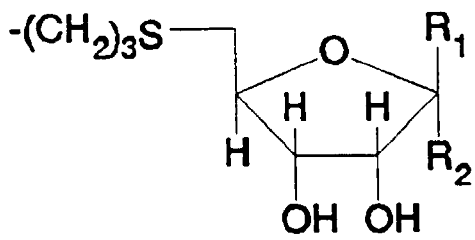

These may be produced for example by reacting a carboxylic acid of formula I (or an anhydride or acid halide thereof) with a polymer having a plurality of pendant amines (e.g. polylysine) or an amine of formula I with a polymer having pendent carboxyl groups.

In the kits, all or some of the reagents may be present in dry form, as may the format/matrix for processing the reactions of the reaction mixture. Similarly the kit may, as indicated, include, as means for assessing a detectable analyte or derivative, relatively inexpensive spectrometric or colorimetric apparatus, e.g. a light source and detector arrangement preset to detect light intensity at a wavelength characteristic of the detectable analyte, etc. or even a simple colorimetric calibration chart.

The invention will now be described by means of the following non-limiting Examples. The assay of Example 19 is especially preferred.

Example 1

The sample (an aqueous homocysteine solution for calibration or plasma or urine for clinical assay) was pretreated with a reducing agent (e.g. 10mM dithiothreitol). This sample was added, preferably to a final concentration of homocysteine in the range $10^{-6}$-$10^{-5}$ mol/l, to a solution at 37°C comprising rabbit IgG 5 mg/ml, adenosine deaminase 20 mU/ml, nucleoside phosphorylase 20 mU/ml, xanthine oxidase 20 mU/ml, horseradish peroxidase 500 mU/ml, 10 mmol/l dithiothreitol, 100 mmol/l sodium phosphate, pH adjusted to 7.40, and 100 mU S-adenosyl-1-homocysteine hydrolase. Either simultaneously or subsequently, but preferably after 10 minutes incubation to allow conversion of adenosine within the sample, adenosine was added to a final concentration of $5.10^{-6}$ mol/l. UV absorption was measured during a 10 minute period and the response was measured at 292 nm in a kinetic mode, and the $\frac{\Delta A}{\Delta t}$ was calculated.

For clinical tests the homocysteine concentration may be calculated by interpolation into a standard curve produced using the known standards.

Example 2

The sample (as for Example 1) was pretreated with a reducing agent (e.g. 10mM dithiothreitol). This sample was added, preferably to a final concentration of homocysteine in the range $10^{-6}$-$10^{-5}$ mol/l, to a solution at 37°C comprising rabbit IgG 5 mg/ml, adenosine deaminase 20 mU/ml, xanthine oxidase 20 mU/ml, nucleoside phosphorylase 20 mU/ml, horseradish peroxidase 500 mU/ml, 10 mmol/l dithiothreitol and 100 mmol/l sodium phosphate, pH adjusted to 7.40, 20 mU S-adenosyl-1-homocysteine hydrolase. Then, preferably after 10 minutes incubation to allow conversion of adenosine within the sample to occur, S-adenosyl-l-homocysteine was added to a final concentration of $5.10^{-5}$ mol/l, and the UV absorption was measured during a 10 minute period. At 292 nm in a kinetic mode, the $\frac{\Delta A}{\Delta t}$ was calculated.

For clinical tests the homocysteine concentration may be calculated by interpolation into a standard curve produced using the known standards.

Example 3

Assay buffer:

0.1M phosphate buffer pH 7.4, comprising 1 mg/ml rabbit IgG and 10 mmol/l dithiothreitol.

Assay procedure:

To 150 µl assay buffer, 3 mU S-adenosyl-l-homocysteine hydrolase and sample (preferably pretreated as described in Examples 1 and 2) were added. The enzyme reaction was started by addition of adenosine dissolved in assay buffer, to a final concentration of $2.5x10^{-5}$ mol/l. After 10 minutes incubation, 750 µl of a solution -of assay buffer comprising 20 mU adenosine deaminase, 20 mU nucleoside phosphorylase, 20 mU xanthine oxidase and 375 mU of horseradish

peroxidase were added. The UV absorption at 292 nm was measured for 5 minutes in a kinetic mode. The $\frac{\Delta A}{\Delta t}$ is calculated. In parallel, the assay is repeated but without the addition of S-adenosyl-l-homocysteine hydrolase. The difference between the two values of $\frac{\Delta A}{\Delta t}$ is determined and the homocysteine concentration is calculated by interpolation into a standard curve.

Example 4

The assay procedure is performed as described in Example 3 up to the first incubation. Then, after 10 minutes incubation, an assay solution comprising fluorescein-labelled adenosine and monoclonal anti-adenosine antibodies is added. The remaining adenosine and the fluorescein labelled adenosine compete for binding to the antibody. The amount of labelled adenosine bound to the antibodies is assessed by the conventional fluorescence polarisation technique, and the homocysteine concentration is calculated by interpolation into a standard curve.

Example 5

Assay buffer:

0.1M phosphate buffer pH 7.4, comprising 1 mg/ml rabbit IgG and 10 mmol/l dithiothreitol.

SAH-hydrolase solution:

40 mU/ml S-adenosyl-l-homocysteine-hydrolase are dissolved in the assay buffer.

Adenosine solution:

$5x10^{-8}$ mol/ml adenosine are dissolved in the assay buffer.

Adenosine deaminase solution:

200 mU/ml adenosine deaminase dissolved in the assay buffer.

Phenol/nitroprusside solution:

10 mg/ml phenol and 50 µg/ml sodium nitroprusside in water.

Hypochlorite solution:

11 mmol/l NaOCl is dissolved in 125 mM NaOH.

Assay Procedure:

1. 75 µl of the adenosine solution and 75 µl of the SAH-hydrolase solution are mixed with the sample (preferably pretreated as described in Examples 1 to 4), and kept at 37°C for 10 minutes.
2. 100 µl of adenosine deaminase solution is added, and the mixture is kept at 37°C for 5 minutes.
3. 750 µl of phenol/nitroprusside solution and 750 µl hypochlorite solution is added. After 30 minutes in 37°C, the extinction at 628 nm is measured. In parallel the assay is repeated but without the addition of SAH-hydrolase. The difference between the two values for extinction at 628 nm is determined and the homocysteine concentration is calculated by interpolation of this difference into a standard curve produced using known standards.

Example 6

FORMATION OF FLUOROPHORE-LABELLED SAH

A 10 mmol/l solution of SAH in dimethylformamide is prepared and then diluted 1:10 in a 100 mmol/l phosphate buffer pH = 7.5. To this solution fluorescein isothiocyanate is added to a final concentration of 1 mmol/l. After 60 minutes incubation at ambient temperature, the SAH-fluorescein conjugate is purified by HPLC using a Chromasil C-18 column at 260 nm using a gradient mixture of 25 mM ammonium acetate (pH 7.0) and methanol.

Example 7

FORMATION OF ANTI-SAH-ANTIBODIES

a) Formation of antigen: To a 1 mmol/l solution of SAH in 25 mmol/l phoshpate buffer pH = 7.4, with 125 mmol/l NaCl, bovine serum albumin is added to a final concentration of 5 mg/ml. To this mixture, bis(sulfosuccinimidyl)suberate is added to a final concentration of 1 mmol/l, and the mixture is left to react for 60 minutes. (The pH is kept low in this conjugation reaction to stimulate conjugation at the adenosyl amine rather than at the homocysteine amine function). The proteinaceous fraction of the solution - which also comprises the conjugates between BSA and SAH - is isolated by size exclusion chromatography using a Pharmacia Superose 12 column with phosphate buffered saline as eluant.

b) Formation of hybridomas: With the antigen described, hybridomas are formed according to the procedure described by James W. Gooding in "Monoclonal antibodies: Principle and Practice", Academic Press, London, 1983, Chapter 3.

c) Selection of hybridomas:

(i) Hybridomas producing anti-SAH-antibodies are identified as follows: The IgG content of the supernate of the hybridomas is measured by conventional ELISA technique. Then supernate is mixed in a cuvette with a buffer comprising 50 mmol/l phosphate, 120 mmol/l NaCl, pH = 7.4, and 0.1 mg rabbit IgG per ml, to a final concentration of 0.1 µmol/l mouse IgG. Fluorescein labelled SAH, formed according to Example 6 above, is added to a final concentration of 0.02 µmol/l. After 10 minutes incubation, the degree of polarization is measured and with a spectrofluorometer equipped with a fluorescence polarization unit by measuring

A = the fluorescence intensity when the plane of polarization of incident light is parallel to the polarization plane of the filter used for filtration of the emitted light,

B = the fluorescence intensity when the plane of the polarization of incident light is perpendicular to the polarization plane of the filter used for filtration of the emitted light, and using an excitation wavelength of 494 nm and detecting the emitted light at 517 nm.

The degree of polarization is calculated as

$$(A-B) / (A+B)$$

A low degree of polarization indicates that the monoclonal mouse antibodies do not bind SAH.

(ii) From the hybridomas selected according to (i), the hybridomas producing antibodies reactive to adenosine and/or homocysteine are identified as follows: Monoclonal anti-SAH-antibodies from the hybridoma supernate is coated onto microtitre well surfaces as described in Example 9 below. Carbon-14 labelled adenosine (or carbon-14 labelled homocysteine), available from Amersham Ltd, UK, in a buffer comprising 25 mmol/l phosphate, 120 mmol/l NaCl and 1 mg/ml of rabbig IgG and having pH = 7.4 is added to the microtitre wells. After 60 minutes incubation, the wells are washed 3 times with the same buffer (not of course containing the adenosine or homocysteine). High values of radioactivity retained in the wells indicate that the hybridomas produce antibodies which bind to adenosine (or homocysteine) on its own. These antibodies are not used in the assay.

(iii) Production of monoclonal IgG: Hybridomas which produce antibodies which bind to SAH according to (i) above but which do not bind to adenosine or homocysteine according to (ii) above are selected for monoclonal IgG production. The hybridomas selected are used for production of ascites in mice or production of cell cultures in vitro, according to conventional techniques. The monoclonal antibodies are furthermore isolated from the ascites or cell culture media according to conventional techniques. See James W. Gooding "Monoclonal antibodies: Principle and practice", Academic Press, London, 1983.

Example 8

FLUORESCENCE POLARIZATION IMMUNOASSAY OF L-HOMOCYSTEINE

Enzyme solution: 50 mmol/l phosphate buffer pH = 7.4 comprising 0.2 mg/ml rabbit IgG, 120 mmol/l NaCl, 10 mmol/l dithiothreitol, 10 U/l S-adenosyl-l-homocysteine-hydrolase and 0.1 mmol/l adenosine.

Fluorescein-labelled SAH solution: SAH conjugated with fluorescein, formed according to Example 6, is dissolved to a final concentration of 1 µmol/l in 50 mmol/l phosphate buffer pH = 7.4 with 125 mmol/l NaCl and 0.2 mg/ml rabbit IgG.

Antibody solution: Monoclonal anti-SAH antibodies (not reactive with adenosine and preferably also not reactive with homocysteine), e.g. formed according to Example 7, dissolved to a final concentration of 0.1 µmol/l in 50 mmol/l phosphate buffer pH = 7.4 with 125 mmol/l NaCl and 0.2 mg/ml rabbit IgG.

Assay performance: In a cuvette, 15 µl plasma (initially a series of samples of known homocysteine content) is mixed with 100 µl enzyme solution and kept at 37 degrees Celsius for 15 minutes. 100 µl of the solution of fluorescein-labelled SAH is added, followed by the addition of 1.0 ml of the antibody solution. With a spectrofluorometer equipped

with a fluorescence polarization unit, the degree of polarization is measured as described in Example 7(c)(i) above and is plotted against the homocysteine concentration.

The assay can also be performed using the labelled haptens and antibodies of Examples 11 and 13 or 15 and 17 in place of those of Examples 6 and 7.

Example 9

MICROTITRE ENZYME-LINKED IMMUNOASSAY

(a) The enzyme solution of Example 8 is used.

(b) Solution of peroxidase-labelled SAH: 0.5 mg horseradish peroxidase is dissolved in 1 ml purified water. 200 µl of a solution of 0.02 mol/l sodium periodate is added, the mixture is stirred for 20 minutes at ambient temperature, and dialyzed overnight against a 10 mM sodium acetate buffer pH = 4.4. SAH is added to a final concentration of 0.1 mmol/l and the pH is adjusted to 6.0. The solution is stirred for 4 hours at ambient temperature. 100 µl of freshly prepared 4 mg/ml aqueous solution of sodium borohydride is added, and the solution is incubated at 4 degrees Celsius for 2 hours. The peroxidase and its SAH conjugates are isolated by size exclusion chromatography in a column of Superose 6 (Pharmacia, Sweden).

(c) Anti-SAH-antibodies coated on microtitre wells: Polyclonal sheep IgG, from sheep immunized to mouse IgG, is dissolved to a final concentration of 1 mg/ml in 100 mmol/l borate buffer pH = 9.0. 300 µl of this solution is filled in each of the wells of polystyrene microtitre plates. After 120 minutes incubation at 37 degrees Celsius, the wells are washed 5 times with phosphate buffered saline. Thereafter, monoclonal mouse IgG anti-SAH-antibodies formed according to Example 7 above, are dissolved in phosphate buffered saline to a final concentration of 50 µg/ml. 200 µl of this monoclonal IgG solution is added to each well and incubated for 120 minutes at 37 degrees Celsius. The wells are then washed 5 times with phosphate buffered saline solution containing 0.1 mg/ml of rabbig IgG.

(d) Assay performance A 25 µl plasma sample (initially a series of samples of known homocysteine concentration) is mixed with 500 µl of the enzyme solution and kept at 37 degrees Celsius for 15 minutes. 50 µl of the peroxidase-labelled SAH solution is added, and, following mixing, 250 µl of this mixture is added to a well in the anti-SAH-antibody coated microtitre wells produced according to (c) above all buffered to pH 7.4. After 60 minutes incubation at 37 degrees Celsius, the wells are washed three times with phosphate buffered saline containing 0.1 mg/ml rabbit IgG. 100 µl of a 1 mg/ml solution of ortho-phenylenediamine in a 0.1 mol/l citrate buffer pH = 6.0 containing 0.015% hydrogen peroxide is added to each well. After 10-30 minutes the light absorbance of each well is read at 450 nm. The absorbance is plotted against the homocysteine concentration.

Example 10

Hapten production

3-S-(1-Anhydro-D-ribofuranosyl)-thiopropyl amine

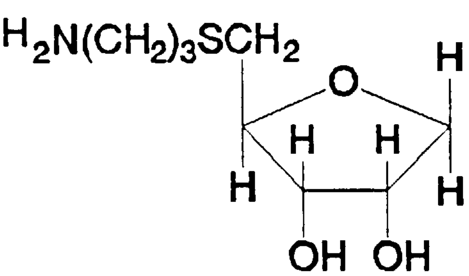

(a) Activated Hydroxy-Protected mercaptan (Compound (8) in Scheme (E) above)

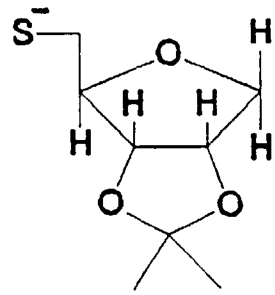

One equivalent of methyl β-D-ribofuranoside (Compound (1) above which is commercially available) is reacted with a mixture of 5 equivalents of trimethylsilylmethane sulphonate and one equivalent of boron trifluoride etherate using the procedure of Jun et al. (Carb. Res. 163: 247-261 (1987)). 0.5 equivalents of the 1-anhydro-D-ribose product (Compound (2)) is added in finely powdered form, in small portions, under continuous stirring to an acetone/sulphuric acid mixture produced by adding 6.3 ml of concentrated sulphuric acid slowly to 100 ml of freshly distilled acetone in an ice bath. The ice bath is removed and reaction is allowed to continue at ambient temperature for 8 hours. The solid white crystalline mass obtained is dissolved in chloroform, washed with aqueous sodium hydroxide, dilute hydrochloric acid and finally water, dried and evaporated down to yield the 2,3-isopropylidene-D-ribonic derivative of 1-anhydro-D-ribose (Compound (2)). One equivalent of this and two equivalents of carbon tetrabromide are dissolved in dry ether and cooled on ice. With constant stirring and cooling on ice, two equivalents of triphenylphosphine are added slowly. The ice bath is removed and the mixture is allowed to warm up to ambient temperature letting the reaction take place and causing hydrogen bromide to evolve smoothly. After the reaction is complete, excess reagent is quenched with the addition of methanol. The bromide derivative (Compound (6)) is isolated by filtration and evaporation down of the filtrate. To the bromide derivative is added thiourea (one equivalent) dissolved in warm water and diluted with rectified spirit. The mixture is refluxed and shaken well periodically, this continuing until about 30 minutes after the bromide derivative dissolves. The reaction mixture is cooled on ice and filtered to yield a solid which is treated with alkaline water producing the hydroxy-protected mercaptan (Compound (7)) in the organic phase. This is converted to the activated form (Compound (8)) by treatment with methoxide in methanol. This is then reacted further as described below.

(b) 3-S-(1-Anhydro-D-ribofuranosyl)thiopropylamine

One equivalent of the compound of Example 10(a) (Compound (8)) freshly prepared is reacted with one equivalent of acrylonitrile to yield a thioether (Compound (9)). This is then reduced by treatment with $LiAlH_4$ in dry ether and the free deprotected amine (Compound (10)) is isolated by treatment with aqueous hydrochloric acid.

The corresponding aminopropylthioethers in which $R_1$ and $R_2$ are other than hydrogen are produced analogously, e.g. using the commercially available compounds (1) and (3) as starting materials.

Example 11

Hapten labelling

A 50 mmol/l solution of the compound of Example 10 in dimethylformamide (DMF) is diluted 1:5 (by volume) in 0.1M bicarbonate solution (pH 9.2). To this solution, fluorescein isothiocyanate is added to a final concentration of 12 mmol/l. After 60 minutes incubation at ambient temperature, the fluorescein conjugate of the compound of Example 10 is purified by RPC using a Kromasil 100 Å C-18 column and a gradient mixture of 20 mM ammonium acetate (pH 7.0) and methanol.

Example 12

Antigen preparation

To a 1 mmol/l solution of the compound of Example 10 in 50 mmol/l phosphate, 125 mmol/l NaCl (pH 7.4) buffer, bovine serum albumin (BSA) is added to a final concentration of 5 mg/ml. To this mixture, bis(sulfosuccinimidyl)suberate is added to a final concentration of 1.2 mmol/l and the mixture is left to react for 60 minutes. The proteinaceous fraction, which includes the hapten-BSA conjugate, is isolated by size exclusion chromatography using a Pharmacia Superose

12 column with phosphate buffered saline as eluant.

Example 13

Antibody preparation

Antibodies to the compound of Example 10 are prepared analogously to Example 7 above using the antigen of Example 12. Antibodies not reactive with SAH and antibodies reactive with adenosine are rejected as preferably are antibodies reactive with homocysteine.

Example 14

Hapten production

N-Hydroxy succinimidyl 3-S-(1-Anhydro-D-ribofuranosyl)thiobutanoate

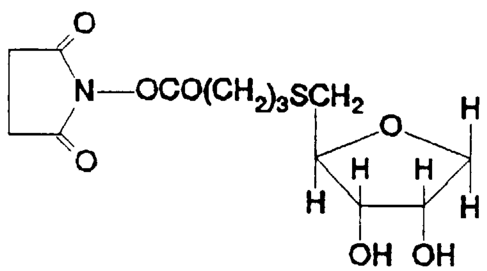

One equivalent of the compound of Example 10(a), freshly prepared, is reacted with 1 equivalent of ethyl 4-bromobutyrate to yield the ester compound (11). This is hydrolysed to the free acid by basic hydrolysis with aqueous sodium hydroxide in dioxane and deprotected by treatment with aqueous hydrochloric acid to yield the unprotected free acid (Compound (12)). One equivalent of this is mixed with two equivalents of N-hydroxysuccinimide in ice-cold dimethylformamide and to this is added 1.2 equivalents of dicyclohexylcarbodiimide under constant stirring. The reaction is allowed to proceed at ambient temperature for 18 hours, whereafter the mixture is cooled and ice-cold ether is added. The precipitated NHS-ester (Compound (13)) is recrystallized from DMF/ether, dried and then stored at 4°C over a desiccant.

The corresponding NHS-esters in which $R_1$ and $R_2$ are other than hydrogen are produced analogously, e.g. using the commercially available compounds (1) and (3) as starting materials.

Example 15

Hapten labelling

A 50 mmol/l solution of the compound of Example 14 in DMF is diluted 1:5 (by volume) in 0.1 M bicarbonate buffer (pH 9.2). To this solution, 5-aminoacetamidofluorescein (fluoresceinyl glycine amide) as added to a final concentration of 12 mmol/l. After 60 mintues incubation at ambient temperature, the fluorescein conjugate of 3-S-(1-anhydro-D-ribofuranosyl)thiobutanoic acid is purified by RPC using a Kromasil 100 Å, C-18 column and a gradient mixture of 20 mM ammonium acetate (pH 7.0) and methanol.

Example 16

Antigen preparation

To a solution of BSA (5 mg/l in 50 mmol/l phosphate, 125 mmol/l NaCl (pH 7.4) buffer), the compound of Example 14 is added to a final concentration of 1 mmol/l. The mixture is left to react for 60 minutes and the proteinaceous fraction, which contains the BSA-hapten conjugate, is isolated by size exclusion chromatogrpahy using a Pharmacia Superose 12 column with phosphate buffered saline as eluant.

Example 17

Antibody preparation

Antibodies to the compound of Example 14 are prepared analogously to Example 7 above using the antigen of Example 12. Antibodies not reactive with SAH and antibodies reactive with adenosine are rejected as preferably are antibodies reactive with homocysteine.

Example 18

Fluorescence Polarization Immunoassay

Enzyme solution:

50 mmol/l phosphate buffer (pH 7.4) containing 4 mg/ml casein, 120 mmol/l NaCl, and 10 U/l S-adenosyl-L-homocysteine-hydrolase.

Dithiothreitol (DTT)-solution:

Dithiothreitol is dissolved in water to a concentration of 50 mmol/l and adjusted to pH 3.0 with HCl.

Adenosine-solution:

1.8 mmol/l adenosine in 50 mmol/l phosphate buffer (pH 7.4).

Fluorescein-labelled SAH solution:

50 mmol/l phosphate buffer (pH 7.4) containing SAH conjugated to fluorescein, formed according to Example 6.

Antibody solution:

Monoclonal anti-SAH antibodies (e.g. according to Example 7) dissolved to a final concentration of 0.1 µmol/l in 50 mmol/l phosphate buffer (pH 7.4) containing 120 mmol/l NaCl and 1 mg/ml casein.

Assay performance

Step 1:

In a cuvette, 15µl of sample, 10 µl enzyme solution and 10 µl adenosine solution are mixed with 10 µl of the acidic DTT-solution and kept at 37°C for 15 minutes.

Step 2:

To the cuvette, 100 µl of the fluorescein-labelled SAH solution and 1.0 ml of the antibody solution are added.

With a spectrofluorometer equipped with a fluorescence polarization unit, the degree of polarization is measured as described in Example 7(c) (i) above and is plotted against the homocysteine concentration.

Example 19

Fluorescence polarization immunoassay

Enzyme solution:

50 mmol/l phosphate buffer (pH 7.4) containing 1 mg/ml casein, 120 mmol/l NaCl, and 10 U/l S-adenosyl-L-homocysteine-hydrolase.

Dithiothreitol (DTT)-solution:

Dithiothreitol is dissolved in water to a concentration of 50 mmol/l and adjusted to pH 3.0 with HCl.

Fluorescein-labelled SAH solution/adenosine-solution:

50 mmol/l phosphate buffer (pH 7.4) containing 10 µmol/l SAH conjugated to fluorescein, formed according to Example 6, and 1.8 mmol/l adenosine.

Antibody solution:

Monoclonal anti-SAH antibodies (e.g. according to Example 7) dissolved to a final concentration of 0.1 µmol/l in 50 mmol/l phosphate buffer (pH 7.4) containing 120 mmol/l NaCl and 1 mg/ml casein.

Assay performance

In a cuvette, 10 µl plasma, 100 µl enzyme solution and 10 µl labelled SAH/adenosine-solution are mixed with 30 µl of the acidic DTT-solution and kept at 37°C for 15 minutes.

After incubation, 1.0 ml of the antibody solution is added. With a spectrofluorometer equipped with a fluorescence polarization unit, the degree of polarization is measured as described in Example 7(c) (i) above and is plotted against the homocysteine concentration.

The assays of Examples 18 and 19 can also be performed using the labelled haptens and antibodies of Examples 11 and 13 or 15 and 17 in place of those of Examples 6 and 7.

Example 20

Luminescence assay

Assay buffer I:

50 mM Pipes-buffer (pH 6.6) containing 1 mg/ml casein, 10 mM DTT, 0.5 mM $Mgcl_2$, and 30 mM KCl.

Assay buffer II:

40 mM Hepes-buffer (pH 7.75), 4 mmol/l EDTA, 20 mM magnesium chloride and 0.36 mmol/l DTT.

Assay buffer III:

40 mM Hepes-buffer (pH 7.75) containing 1.6 µg/ml luciferase (from Photinus pyralis), 700 µmol/l D-luciferin, 20 mmol/l magnesium chloride, 4 mmol/l EDTA, 0.36 mmol/l DTT and 0.3 mmol/l AMP.

Assay procedure:

To 130 µl of Assay buffer I is added 3 U of S-adenosyl-1-homocysteine hydrolase, 20 µl sample is added to this mixture and it is incubated for 15 minutes at 37 degrees Celsius. Thereafter, adenosine dissolved in Assay buffer I to a final concentration of $5x10^{-6}$ mol/l is added. After 5 minutes incubation at 37°C, 750 µl of Assay buffer I containing $0.7x10^{-5}$ mol/l ATP and 1 mU adenosine kinase are added, and the resulting solution is further incubated at 37°C for 5 minutes. This solution is diluted 1:100 (by volume) with Assay buffer II and 500 µl of this diluted solution is immediately added to 500 µl of Assay buffer III. Both Assay buffers II and III were equilibrated to ambient temperature (21°C). The luminescence produced is read in a photometer at 550 nm.

In parallel, an assay with no S-adenosyl-1-homocysteine hydrolase present is run. For clinical tests the homocysteine concentrations may be calculated by interpolation into a standard curve the difference in luminescence produced with and without S-adenosyl-1-homocysteine hydrolase present.

Example 21

Polyclonal antibody preparation

Rabbit polyclonal antibodies to the antigens of Examples 12 and 16 are raised according to the protocol issued by the Dako Corporation, Copenhagen, Denmark. Polyclonal IgG is purified from the collected antiserum according to the same protocol. The polyclonal antibodies are purified from antibodies reactive with adenosine and homocysteine residues per se by passing the antibodies through Racti-Gel columns with immobilized adenosine and homocysteine residues (Gel and protocol as provided by Pierce Chemical Company, Belfium). Antibodies unreactive with SAH are also rejected. The selected antibodies can be used in the assays of the earlier Examples.

## Claims

**1.** A method for assaying homocysteine in a sample, said method comprising the steps of (a) contacting said sample with the enzyme S-adenosyl homocysteine hydrolase and at least one of (i) S-adenosyl homocysteine and (ii) adenosine or an adenosine analogue, and (b) without chromatographic separation assessing a non-labelled analyte selected from adenosine, said adenosine analogue and S-adenosyl homocysteine.

**2.** A method as claimed in claim 1 wherein said sample is contacted with an antibody to said analyte and with a hapten for said antibody other than said non-labelled analyte, and wherein assessment of said analyte is effected indirectly by assessment of said hapten either bound or not bound to said antibody.

**3.** A method as claimed in claim 2 wherein said hapten is a polyhapten.

**4.** A method as claimed in claim 2 wherein said hapten is a labelled molecule having an epitopic structural unit which is also present in said non-labelled analyte.

**5.** A method as claimed in any one of claims 2 to 4 wherein said antibody is a monoclonal antibody.

**6.** A method as claimed in any one of claims 2 to 5 wherein said antibody is a carrier matrix bound antibody.

**7.** A method as claimed in claim 1 wherein said sample is contacted with a second enzyme serving to convert said analyte and assessment of said analyte is effected indirectly by assessment either of a substrate of said second enzyme other than said analyte or of a product of enzymic conversion of said analyte by said second enzyme.

**8.** A method as claimed in any one of claims 1 to 7 wherein said analyte is adenosine.

**9.** A method as claimed in claim 8 wherein said sample is further contacted with an adenosine converting enzyme other than S-adenosyl homocysteine hydrolase, said analyte is adenosine, and assessment of adenosine is effected indirectly by assessment of a co-substrate or a reaction product of adenosine conversion by said adenosine converting enzyme.

**10.** A method as claimed in claim 9 wherein said adenosine converting enzyme is adenosine kinase.

**11.** A method as claimed in claim 9 wherein said adenosine converting enzyme is adenosine deaminase.

**12.** A method as claimed in any one of claims 1 to 7 wherein said analyte is S-adenosyl homocysteine.

**13.** A method as claimed in any one of claims 1 to 12 wherein said sample is a blood, plasma or urine sample pretreated with a disulphide bond cleaving reducing agent.

**14.** A method as claimed in any one of claims 1 to 13 wherein the assessment of said analyte is effected photometrically.

**15.** A method as claimed in claim 14 wherein said assessment is effected spectrometrically or colorimetrically.

**16.** A method as claimed in claim 14 wherein said assessment is effected turbidimetrically or nephelometrically.

**17.** A method as claimed in claim 14 wherein said assessment is effected using fluorescence polarization detection.

**18.** A method as claimed in any one of claims 1 to 7, 12 to 15 and 17 wherein assessment of said analyte is effected indirectly by detection of a chromophore or fluorophore on labelled S-adenosyl homocysteine or on a labelled furanose 6-thioether.

**19.** A method as claimed in claim 1 wherein a sample of blood, plasma or urine is treated with a reducing agent; the sample is contacted with adenosine and S-adenosyl-homocysteine hydrolase; the resultant mixture is incubated and then contacted with ATP and adenosine kinase and the mixture is incubated for at least one minute; the resulting mixture is contacted with luciferin and luciferase and light generated is detected.

**20.** A method as claimed in claim 1 wherein a sample of blood, plasma or urine is treated with a reducing agent; the sample is contacted with adenosine and S-adenosyl-homocysteine hydrolase; the resultant mixture is incubated and then contacted with adenosine deaminase, nucleoside phosphorylase, xanthine oxidase and a peroxidase and the UV absorption of the mixture is assessed.

**21.** A method as claimed in claim 1 wherein said sample is contacted with adenosine and an S-adenosyl-homocysteine-hydrolase; the resultant mixture is incubated and then contacted with a fluorophore labelled S-adenosyl-homocysteine or a fluorophore labelled furanose 6-thioether; the resultant mixture is contacted with a monoclonal anti-S-adenosyl-homocysteine antibody; and the antibody-bound or non-antibody bound fluorophore labelled compound is assessed by fluorescence polarization whereby to provide an indication of the initial homocysteine level of the sample.

**22.** A method as claimed in claim 1 wherein said sample is contacted with adenosine and an S-adenosyl-homocysteine hydrolase; the resultant mixture is incubated and then contacted with a labelled S-adenosyl-homocysteine or a labelled furanose 6-thioether; the resultant mixture is contacted with carrier matrix bound monoclonal anti-S-adenosyl-homocysteine antibody; the antibody carrier matrix is washed; and the antibody-bound labelled compound is photometrically assessed whereby to provide an indication of the initial homocysteine level of the sample.

**23.** A method as claimed in claim 22 wherein the label of the antibody bound labelled compound is reacted to generate a chromophore which is then photometrically assessed.

**24.** A method as claimed in claim 3 wherein assessment is affected by assessment of the precipitation or agglutination of antibody:polyhapten conjugates.

**25.** An analytical kit for use in the assay of homocysteine in a sample by a method as claimed in claim 1, said kit comprising: S-adenosyl-homocysteine hydrolase; a substrate selected from adenosine, adenosine analogues and S-adenosyl homocysteine; a signal forming agent; and, optionally, means for signal assessment.

**26.** A kit as claimed in claim 25 comprising: S-adenosyl-homocysteine hydrolase; as a signal forming substrate for said enzyme, labelled S-adenosyl- homocysteine; an optionally carrier matrix bound anti-S-adenosyl homocysteine antibody; and, optionally, as said means for signal assessment, means for photometrically assessing said labelled S-adenosyl homocysteine or the detectable derivative thereof whereby to provide an indication of the homocysteine content of the sample.

**27.** A kit as claimed in claim 25 comprising: S-adenosyl-homocysteine hydrolase; adenosine; an adenosine converting enzyme other than S-adenosyl homocysteine hydrolase; optionally, an adenosine co-substrate for said adenosine converting enzyme; and as said means for signal assessment, means for generating a photometrically detectable response from said co-substrate or from a product of enzymic conversion of adenosine by said adenosine converting enzyme.

**28.** A kit as claimed in claim 27 wherein said adenosine converting enzyme is adenosine kinase and wherein said means for generating comprises ATP, luciferin and a luciferase.

**29.** A kit as claimed in claim 27 wherein said adenosine converting enzyme is adenosine deaminase and said means for generating comprises nucleoside phosphorylase, xanthine oxidase and a peroxidase.

**30.** A kit as claimed in claim 27 comprising: S-adenosyl-homocysteine hydrolase; adenosine; an optionally matrix

particle bound anti-S-adenosyl-homocysteine antibody; a polyhapten for said antibody; and, optionally, as said means for signal assessment, means for photometrically assessing agglutination or precipitation of antibody:polyhapten complexes.

**31.** A method as claimed in claim 2 wherein as said hapten is used a compound of formula I

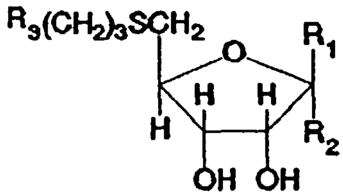

(I)

(wherein $R_1$ and $R_2$ which may be the same or different denote hydrogen atoms or $OR_4$ groups or $R_1$ and $R_2$ together denote an oxygen atom, $R_3$ denotes an amino or carboxyl group, and $R_4$ denotes a $C_{1-6}$aliphatic group) or a salt or ester thereof.

**32.** A method as claimed in claim 4 wherein as said hapten is used a labelled furanose 6-thioether, wherein the labelled moiety comprises a chromophore or fluorophore or a radioactive atom, and wherein the thioether moiety comprises a trimethylenethio moiety.

**33.** A method as claimed in claim 32 wherein as said labelled thioether is used a labelled compound of formula I as defined in claim 31.

**34.** A method as claimed in claim 1 wherein as said enzyme is used inactivated SAH-hydrolase activated by contact with a reducing agent.

**35.** A kit as claimed in claim 25 comprising in a first compartment inactive SAH-hydrolase and in a second compartment a reducing agent, whereby to produce on admixture of the contents of said first and second compartments activated SAH-hydrolase.

**36.** A kit as claimed in claim 35 wherein said second compartment comprises dithiothreitol in an acid medium.

**Patentansprüche**

**1.** Verfahren zur Bestimmung des Homocysteingehaltes einer Probe, wobei man (a) die Probe mit dem Enzym S-Adenosylhomocysteinhydrolase und mit wenigstens einer Verbindung, ausgewählt unter (i) S-Adenosylhomocystein und (ii) Adenosin oder einem Adenosinanalogen, in Kontakt bringt und (b) einen nicht-markierten Analyten, der ausgewählt ist unter Adenosin, dem Adenosinanalogen und S-Adenosylhomocystein, ohne chromatographische Trennung bestimmt.

**2.** Verfahren nach Anspruch 1, wobei die Probe mit einem Antikörper gegen den Analyten und mit einem Hapten für den Antikörper in Kontakt gebracht wird, das von dem nicht-markierten Analyten verschieden ist, und wobei die Bestimmung des Analyten indirekt durch Bestimmung des an den Antikörper gebundenen bzw. nicht gebundenen Haptens erfolgt.

**3.** Verfahren nach Anspruch 2, wobei das Hapten ein Polyhapten ist.

**4.** Verfahren nach Anspruch 2, wobei das Hapten ein markiertes Molekül ist, welches eine auch in dem nicht-markierten Analyten vorhandene Epitop-Struktureinheit aufweist.

**5.** Verfahren nach einem der Ansprüche 2 bis 4, wobei der Antikörper ein monoklonaler Antikörper ist.

**6.** Verfahren nach einem der Ansprüche 2 bis 5, wobei der Antikörper ein an eine Trägermatrix gebundener Antikörper ist.

**7.** Verfahren nach Anspruch 1, wobei die Probe mit einem zweiten Enzym in Kontakt gebracht wird, das zur Umwandlung des Analyten dient, und wobei die Bestimmung des Analyten indirekt, durch Bestimmung eines von dem Analyten verschiedenen Substrates des zweiten Enzyms oder eines Produktes der enzymatischen Umwandlung des Analyten durch das zweite Enzym, erfolgt.

**8.** Verfahren nach einem der Ansprüche 1 bis 7, wobei der Analyt Adenosin ist.

**9.** Verfahren nach Anspruch 8, wobei die Probe zusätzlich mit einem von S-Adenosylhomocysteinhydrolase verschiedenen, Adenosin-umwandelnden Enzym in Kontakt gebracht wird, und wobei es sich bei dem Analyten um Adenosin handelt und die Bestimmung des Adenosins indirekt, durch Bestimmung eines Co-Substrates oder eines Reaktionsproduktes der Adenosinumwandlung durch das Adenosin-umwandelnde Enzym, erfolgt.

**10.** Verfahren nach Anspruch 9, wobei das Adenosin-umwandelnde Enzym Adenosinkinase ist.

**11.** Verfahren nach Anspruch 9, wobei das Adenosin-umwandelnde Enzym Adenosindeaminase ist.

**12.** Verfahren nach einem der Ansprüche 1 bis 7, wobei der Analyt S-Adenosylhomocystein ist.

**13.** Verfahren nach einem der Ansprüche 1 bis 12, wobei die Probe eine Blut-, Plasma- oder Urinprobe ist, die mit einem Disulfidbindungen spaltenden Reduktionsmittel vorbehandelt wurde.

**14.** Verfahren nach einem der Ansprüche 1 bis 13, wobei die Bestimmung des Analyten photometrisch erfolgt.

**15.** Verfahren nach Anspruch 14, wobei die Bestimmung spektrometrisch oder colorimetrisch erfolgt.

**16.** Verfahren nach Anspruch 14, wobei die Bestimmung turbidimetrisch oder nephelometrisch erfolgt.

**17.** Verfahren nach Anspruch 14, wobei die Bestimmung mittels Fluoreszenzpolarisationsdetektion erfolgt.

**18.** Verfahren nach einem der Ansprüche 1 bis 7, 12 bis 15 und 17, wobei die Bestimmung des Analyten indirekt, durch Detektion eines Chromophors oder Fluorophors auf markiertem S-Adenosylhomocystein oder auf einem markierten Furanose-6-thioether, erfolgt.

**19.** Verfahren nach Anspruch 1, wobei man eine Blut-, Plasma- oder Urinprobe mit einem Reduktionsmittel behandelt; die Probe mit Adenosin und S-Adenosylhomocysteinhydrolase in Kontakt bringt; das resultierende Gemisch inkubiert und dann mit ATP und Adenosinkinase in Kontakt bringt und das Gemisch wenigstens eine Minute inkubiert; das resultierende Gemisch mit Luciferin und Luciferase in Kontakt bringt und das dabei erzeugte Licht detektiert.

**20.** Verfahren nach Anspruch 1, wobei man eine Blut-, Plasma- oder Urinprobe mit einem Reduktionsmittel behandelt; die Probe mit Adenosin und S-Adenosylhomocysteinhydrolase in Kontakt bringt; das resultierende Gemisch inkubiert und dann mit Adenosindeaminase, Nucleosidphosphorylase, Xanthinoxidase und einer Peroxidase in Kontakt bringt und die UV-Absorption des Gemisches bestimmt.

**21.** Verfahren nach Anspruch 1, wobei man die Probe mit Adenosin und einer S-Adenosylhomocysteinhydrolase in Kontakt bringt; das resultierende Gemisch inkubiert und dann mit einem Fluorophor-markierten S-Adenosylhomocystein oder einem Fluorophor-markierten Furanose-6-thioether in Kontakt bringt; das resultierende Gemisch mit einem monoklonalen Anti-S-Adenosylhomocystein-Antikörper in Kontakt bringt; und die an den Antikörper gebundene bzw. nicht an den Antikörper gebundene, Fluorophor-markierte Verbindung durch Fluoreszenzpolarisation bestimmt, um einen Hinweis auf den anfänglichen Homocysteingehalt der Probe zu erhalten.

**22.** Verfahren nach Anspruch 1, wobei man die Probe mit Adenosin und einer S-Adenosylhomocysteinhydrolase in Kontakt bringt; das resultierende Gemisch inkubiert und dann mit einem markierten S-Adenosylhomocystein oder einem markierten Furanose-6-thioether in Kontakt bringt; das resultierende Gemisch mit einem an eine Trägermatrix gebundenen monoklonalen Anti-S-Adenosylhomocystein-Antikörper in Kontakt bringt; die Antikörper-Trägermatrix wäscht; und die an den Antikörper gebundene, markierte Verbindung photometrisch bestimmt, um einen Hinweis auf den anfänglichen Homocysteingehalt der Probe zu erhalten.

**23.** Verfahren nach Anspruch 22, wobei die Markierung der an den Antikörper gebundenen, markierten Verbindung zu einem Chromophor umgesetzt wird, der dann photometrisch bestimmt wird.

**24.** Verfahren nach Anspruch 3, wobei die Bestimmung durch Bestimmung der Präzipitation oder Agglutination von Antikörper:Polyhapten-Konjugaten erfolgt.

**25.** Analytischer Kit zur Verwendung für den Homocystein-Assay in einer Probe durch das Verfahren gemäß Anspruch 1, wobei der Kit umfaßt: S-Adenosylhomocysteinhydrolase; ein Substrat, das ausgewählt ist unter Adenosin, Adenosinanaloga und S-Adenosylhomocystein; ein Signal-erzeugendes Mittel; und gegebenenfalls Mittel zur Signalbestimmung.

**26.** Kit nach Anspruch 25, umfassend: S-Adenosylhomocysteinhydrolase; markiertes S-Adenosylhomocystein als ein Signal erzeugendes Substrat für das Enzym; einen gegebenenfalls an eine Trägermatrix gebundenen Anti-S-Adenosylhomocystein-Antikörper; und gegebenenfalls, als Mittel zur Signalbestimmung, Mittel zur photometrischen Bestimmung des markierten S-Adenosylhomocysteins oder des detektierbaren Derivates davon, um einen Hinweis auf den Homocysteingehalt der Probe zu erhalten.

**27.** Kit nach Anspruch 25, umfassend: S-Adenosylhomocysteinhydrolase; Adenosin; ein von S-Adenosylhomocysteinhydrolase verschiedenes, Adenosin-umwandelndes Enzym; gegebenenfalls ein Adenosin-Cosubstrat für das Adenosin-umwandelnde Enzym; und, als Mittel zur Signalbestimmung, Mittel zur Erzeugung einer photometrisch detektierbaren Antwort des Cosubstrates oder eines Produktes der enzymatischen Umwandlung von Adenosin mit dem Adenosin-umwandelnden Enzym.

**28.** Kit nach Anspruch 27, worin das Adenosin-umwandelnde Enzym Adenosinkinase ist und worin das erzeugende Mittel ATP, Luciferin und eine Luciferase umfaßt.

**29.** Kit nach Anspruch 27, worin das Adenosin-umwandelnde Enzym Adenosindeaminase ist und das erzeugende Mittel Nucleosidphosphorylase, Xanthinoxidase und eine Peroxidase umfaßt.

**30.** Kit nach Anspruch 27, umfassend: S-Adenosylhomocysteinhydrolase; Adenosin; einen gegebenenfalls an Matrixpartikel gebundenen Anti-S-Adenosylhomocystein-Antikörper; ein Polyhapten für den Antikörper; und gegebenenfalls, als Mittel zur Signalbestimmung, Mittel zur photometrischen Bestimmung der Agglutination oder Präzipitation von Antikörper:Polyhapten-Komplexen.

**31.** Verfahren nach Anspruch 2, wobei als Hapten eine Verbindung der Formel I

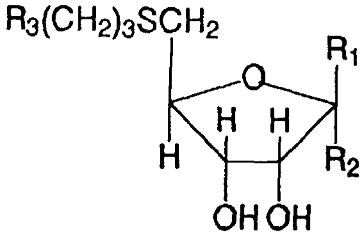

(I)

(worin $R_1$ und $R_2$ gleich oder verschieden sind und für Wasserstoffatome oder $OR_4$-Gruppen stehen, oder $R_1$ und $R_2$ zusammen für ein Sauerstoffatom stehen, $R_3$ für eine Amino- oder Carboxylgruppe und $R_4$ für eine aliphatische $C_{1-6}$-Gruppe steht) oder ein Salz oder Ester davon verwendet wird.

**32.** Verfahren nach Anspruch 4, wobei als Hapten ein markierter Furanose-6-thioether verwendet wird, worin die markierte Einheit einen Chromophor oder Fluorophor oder ein radioaktives Atom umfaßt und worin die Thioethereinheit eine Trimethylenthio-Einheit umfaßt.

**33.** Verfahren nach Anspruch 32, wobei als markierter Thioether eine markierte Verbindung der in Anspruch 31 definierten Formel I verwendet wird.

**34.** Verfahren nach Anspruch 1, wobei als Enzym inaktivierte SAH-Hydrolase verwendet wird, die durch Kontakt mit einem Reduktionsmittel aktiviert wird.

35. Kit nach Anspruch 25, umfassend, in einem ersten Kompartiment, inaktive SAH-Hydrolase und, in einem zweiten Kompartiment, ein Reduktionsmittel, wobei man durch Vermischen der Inhalte des ersten und zweiten Kompartiments aktivierte SAH-Hydrolase erzeugt.

36. Kit nach Anspruch 35, worin das zweite Kompartiment Dithiothreitol in einem sauren Medium umfaßt.

**Revendications**

1. Un procédé pour doser l'homocystéine contenue dans un échantillon, ledit procédé comprenant les étapes de (a) mise en contact de l'échantillon avec l'enzyme S-adénosyl-homocystéine hydrolase et au moins une substance parmi (i) la S-adénosyl-homocystéine et (ii) l'adénosine ou un analogue de l'adénosine, et (b) sans séparation chromatographique, évaluation d'un analyte non marqué sélectionné parmi l'adénosine, ledit analogue de l'adénosine et la S-adénosyl-homocystéine.

2. Un procédé selon la revendication 1, dans lequel ledit échantillon est mis en contact avec un anticorps dudit analyte et avec un haptène dudit anticorps autre que ledit analyte non marqué, et dans lequel le dosage dudit analyte est effectué indirectement par dosage dudit haptène, fixé ou non fixé audit anticorps.

3. Un procédé selon la revendication 2, dans lequel ledit haptène est un polyhaptène.

4. Un procédé selon la revendication 2, dans lequel ledit haptène est une molécule marquée ayant une unité de structure déterminante qui est également présente dans ledit analyte non marqué.

5. Un procédé selon l'une ou l'autre des revendications 2 à 4, dans lequel ledit anticorps est un anticorps monoclonal.

6. Un procédé selon l'une ou l'autre des revendications 2 à 5, dans lequel ledit anticorps est un anticorps lié à une matrice support.

7. Un procédé selon la revendication 1, dans lequel ledit échantillon est mis en contact avec un second analyte servant à convertir ledit analyte et le dosage dudit analyte est effectué indirectement par évaluation soit d'un substrat dudit second enzyme autre que ledit analyte, soit d'un produit de conversion enzymatique dudit analyte par ledit second enzyme.

8. Un procédé selon l'une ou l'autre des revendications 1 à 7, dans lequel ledit analyte est l'adénosine.

9. Un procédé selon la revendication 8, dans lequel ledit échantillon est également mis en contact avec un enzyme de conversion de l'adénosine autre que la S-adénosyl-homocystéine hydrolase, ledit analyte est l'adénosine, et l'évaluation de l'adénosine est effectuée indirectement par évaluation d'un cosubstrat ou d'un produit de réaction de la conversion de l'adénosine par ledit enzyme de conversion de l'adénosine.

10. Un procédé selon la revendication 9, dans lequel ledit enzyme de conversion de l'adénosine est l'adénosine kinase.

11. Un procédé selon la revendication 9, dans lequel ledit enzyme de conversion de l'adénosine est l'adénosine désaminase.

12. Un procédé selon l'une ou l'autre des revendications 1 à 7, dans lequel ledit analyte est la S-adénosyl-homocystéine.

13. Un procédé selon l'une ou l'autre des revendications 1 à 12, dans lequel ledit échantillon est un échantillon de sang, de plasma ou d'urine prétraité avec un agent réducteur provoquant la rupture de la liaison disulfurique.

14. Un procédé selon l'une ou l'autre des revendications 1 à 13, dans lequel l'évaluation dudit analyte est effectuée par photométrie.

15. Un procédé selon la revendication 14, dans lequel ladite évaluation est effectuée par spectrophotométrie ou colorimétrie.

**16.** Un procédé selon la revendication 14, dans lequel ladite évaluation est effectuée par turbidimétrie ou néphélométrie.

**17.** Un procédé selon la revendication 14, dans lequel ladite évaluation est effectuée par détection de polarisation en fluorescence.

**18.** Un procédé selon l'une ou l'autre des revendications 1 à 7, 12 à 15 et 17, dans lequel l'évaluation dudit analyte est effectuée indirectement par détection d'un chromophore ou fluorophore sur la S-adénosyl-homocystéine marquée ou sur un 6-thioéther furanosique marqué.

**19.** Un procédé selon la revendication 1, dans lequel un échantillon de sang, de plasma ou d'urine est traité avec un agent réducteur ; l'échantillon est mis en contact avec de l'adénosine et de la S-adénosyl-homocystéine hydrolase ; le mélange résultant est mis à incuber, puis mis en contact avec de l'ATP et de l'adénosine kinase et le mélange est mis à incuber pendant au moins une minute ; le mélange résultant est mis en contact avec de la luciférine et de la luciférase et la lumière générée est décelée.

**20.** Un procédé selon la revendication 1, dans lequel un échantillon de sang, de plasma ou d'urine est traité avec un agent réducteur ; l'échantillon est mis en contact avec de l'adénosine et de la S-adénosyl-homocystéine hydrolase ; le mélange résultant est mis à incuber, puis mis en contact avec de l'adénosine désaminase, de la nucléoside phosphorylase, de la xanthine oxydase et une peroxydase et l'absorption ultraviolette du mélange est évaluée.

**21.** Un procédé selon la revendication 1, dans lequel ledit échantillon est mis en contact avec de l'adénosine et une S-adénosyl-homocystéine hydrolase ; le mélange résultant est mis à incuber, puis mis en contact avec une S-adénosyl-homocystéine marquée par un fluorophore ou un 6-thioéther furanosique marqué par un fluorophore; le mélange résultant est mis en contact avec un anticorps anti-S-adénosyl-homocystéine monoclonal et le composé marqué par un fluorophore lié à l'anticorps ou non lié à l'anticorps est évalué par polarisation en fluorescence afin de fournir une indication du niveau initial d'homocystéine de l'échantillon.

**22.** Un procédé selon la revendication 1, dans lequel ledit échantillon est mis en contact avec de l'adénosine et une S-adénosyl-homocystéine hydrolase ; le mélange résultant est mis à incuber, puis mis en contact avec une S-adénosyl-homocystéine marquée ou un 6-thioéther furanosique marqué ; le mélange résultant est mis en contact avec un anticorps anti-S-adénosyl-homocystéine monoclonal lié à une matrice support ; la matrice support de l'anticorps est lavée ; et le composé marqué lié à l'anticorps est évalué par photométrie afin de fournir une indication du niveau initial d'homocystéine de l'échantillon.

**23.** Un procédé selon la revendication 22, dans lequel le marqueur du composé marqué lié à un anticorps est mis à réagir pour générer un chromophore qui est ensuite évalué par photométrie.

**24.** Un procédé selon la revendication 3, dans lequel l'évaluation est effectuée par évaluation de la précipitation ou de l'agglutination de conjugués anticorps/polyhaptène.

**25.** Un dispositif d'analyse à utiliser dans le dosage de l'homocystéine contenue dans un échantillon par un procédé selon la revendication 1, ledit dispositif comprenant : de la S-adénosyl-homocystéine hydrolase ; un substrat sélectionné parmi l'adénosine, les analogues de l'adénosine et la S-adénosyl-homocystéine ; un agent formateur de signal ; et, optionnellement, un moyen pour évaluer le signal.

**26.** Un dispositif selon la revendication 25 comprenant ; de la S-adénosyl-homocystéine hydrolase ; comme substrat formateur de signal pour ledit enzyme, une S-adénosyl-homocystéine marquée ; un anticorps anti-S-adénosyl-homocystéine 1 optionnellement lié à une matrice support; et, optionnellement, comme moyen pour évaluer le signal, un moyen pour évaluer par photométrie ladite S-adénosyl-homocystéine marquée ou le dérivé décelable de celle-ci afin de fournir une indication de la teneur en homocystéine de l'échantillon.

**27.** Un dispositif selon la revendication 25 comprenant : de la S-adénosyl-homocystéine hydrolase ; de l'adénosine; un enzyme de conversion de l'adénosine autre que la S-adénosyl-homocystéine hydrolase ; optionnellement, un cosubstrat d'adénosine pour ledit enzyme de conversion de l'adénosine ; et comme moyen d'évaluation du signal, un moyen pour générer une réponse décelable par photométrie à partir dudit cosubstrat ou d'un produit de conversion enzymatique de l'adénosine par ledit enzyme de conversion de l'adénosine.

**28.** Un dispositif selon la revendication 27, dans lequel ledit enzyme de conversion de l'adénosine est l'adénosine kinase et dans lequel ledit moyen de génération comprend l'ATP, la luciférine et une luciférase.

**29.** Un dispositif selon la revendication 27, dans lequel ledit enzyme de conversion de l'adénosine est l'adénosine désaminase et ledit moyen de génération comprend la nucléoside phosphorylase, la xanthine oxydase et une peroxydase.

**30.** Un dispositif selon la revendication 27 comprenant : de la S-adénosyl-homocystéine hydrolase ; de l'adénosine; un anticorps anti-S-adénosyl-homocystéine optionnellement lié à des particules de matrice ; un polyhaptène pour ledit anticorps ; et, optionnellement, comme moyen d'évaluation du signal, un moyen pour évaluer par photométrie l'agglutination ou la précipitation de complexes anticorps/polyhaptène.

**31.** Un procédé selon la revendication 2, dans lequel on utilise comme haptène un composé de la formule I

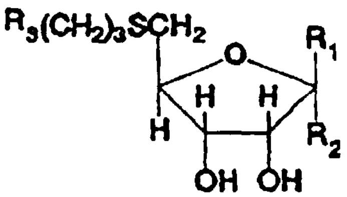

(I)

(dans lequel $R_1$ et $R_2$, qui peuvent être identiques ou différents, indiquent des atomes d'hydrogène ou des groupes $OR_4$ ou $R_1$ et $R_2$ ensemble indiquent un atome d'oxygène, $R_3$ indique un groupe amino ou carboxyle, et $R_4$ indique un groupe aliphatique $C_{1-6}$) ou un sel ou ester de celui-ci.

**32.** Un procédé selon la revendication 4, dans lequel on utilise comme haptène un 6-thioéther furanosique marqué, dans lequel la portion marquée comprend un chromophore ou un fluorophore ou un atome radioactif, et dans lequel la portion sulfure d'alcoyle comprend une portion triméthylènethio.

**33.** Un procédé selon la revendication 32, dans lequel on utilise comme thioéther marqué un composé marqué de la formule I comme défini dans la revendication 31.

**34.** Un procédé selon la revendication 1, dans lequel on utilise comme enzyme une SAH-hydrolase inactivée activée par contact avec un agent réducteur.

**35.** Un dispositif selon la revendication 25 comprenant dans un premier compartiment une SAH-hydrolase inactive et dans un second compartiment un agent réducteur, afin de produire de la SAH-hydrolase activée en mélangeant le contenu desdits premier et second compartiments.

**36.** Un dispositif selon la revendication 35, dans lequel ledit second compartiment comprend du dithiothréitol dans un milieu acide.